(19) Europäisches Patentamt
European Patent Office
Office européen des brevets

(11) **EP 3 687 458 B1**

(12) **FASCICULE DE BREVET EUROPEEN**

(45) Date de publication et mention de la délivrance du brevet:
**08.06.2022   Bulletin 2022/23**

(21) Numéro de dépôt: **18785853.5**

(22) Date de dépôt: **27.09.2018**

(51) Classification Internationale des Brevets (IPC):
**A61F 2/46** *(2006.01)*        **A61B 17/92** *(2006.01)*

(52) Classification Coopérative des Brevets (CPC):
**A61F 2/4657; A61B 17/921; A61F 2/4607;**
A61B 17/92; A61B 2017/00022; A61B 2017/0011;
A61B 2017/00119; A61B 2090/065;
A61F 2002/4658; A61F 2002/4666;
A61F 2002/4681

(86) Numéro de dépôt international:
**PCT/EP2018/076224**

(87) Numéro de publication internationale:
**WO 2019/063673 (04.04.2019 Gazette 2019/14)**

(54) **DISPOSITIF D'INSERTION D'UNIMPLANT CHIRURGICAL**

VORRICHTUNG ZUM EINSETZEN EINES CHIRURGISCHEN IMPLANTATS

DEVICE FOR INSERTING A SURGICAL IMPLANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **29.09.2017  FR 1759136**

(43) Date de publication de la demande:
**05.08.2020   Bulletin 2020/32**

(73) Titulaires:
• **Centre National de la Recherche Scientifique 75016 Paris (FR)**
• **Université Paris XII Val de Marne 94010 Créteil Cedex (FR)**

(72) Inventeurs:
• **HAIAT, Guillaume 94150 Rungis (FR)**
• **ROSI, Giuseppe 75013 Paris (FR)**
• **TIJOU, Antoine 69004 Lyon (FR)**

(74) Mandataire: **Osha Liang 2, rue de la Paix 75002 Paris (FR)**

(56) Documents cités:
EP-A1- 2 923 677          WO-A1-2010/111272
US-A1- 2015 282 856       US-A1- 2017 196 506
US-A1- 2017 196 711

**Description**

DOMAINE TECHNIQUE

L'invention concerne un dispositif pour insérer un implant chirurgical par impaction dans un os récepteur. Ce dispositif comprend un outil de percussion, ou impacteur, adapté pour exercer une force d'impaction (directement ou indirectement) sur l'implant chirurgical afin d'insérer en force celui-ci dans l'os récepteur.

ARRIERE PLAN

Dans le présent exposé, la notion d'implant chirurgical ne se limite pas aux implants chirurgicaux proprement dits mais englobe également les instruments chirurgicaux insérés temporairement dans un os récepteur. Cette notion recouvre, en particulier, l'ensemble des implants de prothèse orthopédique destinés à remplacer une articulation déficiente, notamment les implants de prothèses de hanche, de genoux, d'épaule, de coude, de rachis ou de cheville, et les instruments de pose utilisés pour préparer l'os récepteur à recevoir la prothèse.

L'insertion en force d'un implant chirurgical dans un os récepteur est souvent réalisée par impaction au moyen d'un impacteur, typiquement un marteau. Un ancillaire de pose peut également être utilisé, le praticien utilisant l'impacteur pour frapper l'ancillaire qui transmet la force d'impaction à l'implant.

Au fur et à mesure des impacts, l'implant s'enfonce dans l'os récepteur. Le niveau de contact entre l'implant et l'os qui l'entoure dépend de cet enfoncement. Ce niveau de contact est généralement caractérisé par le ratio BIC (en anglais "BIC ratio" pour "bone to implant contact ratio") qui est le pourcentage de la surface de l'implant au contact de l'os. Plus l'implant est enfoncé dans l'os récepteur, plus le ratio BIC augmente.

Le praticien souhaite généralement suivre l'enfoncement de l'implant dans l'os et déterminer le moment où le niveau de contact entre l'implant et l'os est optimal ou, tout au moins, satisfaisant. Le praticien souhaite également s'assurer du niveau de stabilité primaire dudit implant. La réussite de l'opération chirurgicale d'insertion de l'implant repose à la fois sur un niveau de contact suffisant entre l'implant et l'os récepteur, sur le niveau de stabilité primaire et sur le fait de ne pas endommager et notamment de ne pas induire de fractures ou de microfissures dans l'os récepteur lors de l'insertion. Si l'implant est insuffisamment inséré dans l'os récepteur et/ou insuffisamment stable, il peut s'ensuivre des micromouvements de l'implant pouvant nécessiter une nouvelle intervention chirurgicale.

Un compromis doit donc être trouvé entre un nombre d'impacts suffisamment élevé pour obtenir un niveau de contact os-implant satisfaisant, et suffisamment faible pour ne pas risquer d'endommager l'os récepteur. Or, il est difficile pour le praticien d'évaluer par lui-même avec fiabilité le bon nombre d'impacts. Concrètement, il lui est difficile de savoir précisément quand s'arrêter de frapper l'implant avec l'impacteur.

Dans ce contexte, un but de l'invention est de proposer un dispositif permettant de fournir, durant l'opération chirurgicale, une information fiable sur le niveau de contact entre l'implant et l'os récepteur et de permettre ainsi au praticien de savoir, en temps réel, quand il doit arrêter de frapper l'implant avec l'impacteur.

Le document de brevet FR 3019031 décrit un dispositif d'assistance à la mise en place d'un implant orthopédique, comprenant un impacteur pour impacter une surface d'impact couplée à l'implant et exercer une force d'impaction sur l'implant. L'impacteur a une face de frappe adaptée pour impacter la surface d'impact. Un capteur de force est fixé sur la face de frappe et convertit en un signal électrique exploitable la variation temporelle de la force d'impaction exercée.

Bien qu'il soit généralement satisfaisant, le dispositif du document FR 3019031 a pour inconvénient le fait que le capteur de force doive répondre à plusieurs exigences. Il doit d'abord présenter une résistance mécanique suffisante pour pouvoir supporter les contraintes mécaniques résultant de la force d'impaction exercée. Il doit ensuite résister à des températures élevées car, avant une opération chirurgicale, l'impacteur et son capteur doivent être stérilisés. Lors de la stérilisation, l'impacteur et son capteur sont typiquement portés à haute température, par exemple, en étant maintenus pendant 18 minutes à 134°C sous une pression de 2 bars dans un autoclave. Ces exigences de résistance mécanique et de résistance aux hautes températures limitent significativement le choix parmi les capteurs disponibles sur le marché et les capteurs répondant à ces exigences sont généralement onéreux.

Il existe donc un besoin pour un nouveau type de dispositif qui, tout en répondant aux objectifs généraux poursuivis par l'invention, permette de palier, ou tout au mois de limiter, les inconvénients précités. Le document US2015/282856 A1 décrit un dispositif similaire à celui décrit dans le document de brevet FR 3019031.

PRESENTATION GENERALE

L'invention concerne un dispositif pour insérer en force un implant chirurgical dans un os récepteur, par impaction. Ce dispositif comprend un impacteur adapté pour impacter une surface d'impact couplée audit implant chirurgical et exercer une force d'impaction sur l'implant.

L'impacteur est associé à au moins un capteur apte à mesurer la déformation de l'impacteur, et à fournir un signal de mesure représentant la variation temporelle de cette déformation lors d'un impact.

Le capteur (i.e. ledit au moins un capteur) est relié à une unité de traitement configurée pour calculer, à partir de la variation temporelle de la déformation de l'impacteur lors de l'impact, un indicateur représentatif du niveau de contact entre l'implant et l'os récepteur. La liaison électronique entre le capteur et l'unité de traitement peut être filaire ou non.

La solution proposée repose sur la mise en œuvre d'un ou plusieurs capteurs de déformation associés à l'impacteur et délivrant un signal de mesure, l'enregistrement et l'analyse de ce signal permettant de déterminer un indicateur révélateur du niveau de contact entre l'implant et l'os récepteur. Lorsque plusieurs capteurs sont utilisés, les signaux respectivement délivrés par ces capteurs peuvent être, par exemple, moyennés ou combinés pour obtenir le signal de mesure qui sera analysé et à partir duquel l'indicateur sera calculé.

Comparativement au capteur de force utilisé dans l'art antérieur, le capteur de déformation ne convertit pas en un signal électrique exploitable la force d'impaction exercée par l'impacteur, mais convertit la déformation de l'impacteur en un signal électrique exploitable. Par conséquent, le capteur de déformation n'est pas amené à supporter directement les contraintes mécaniques résultant de la force d'impaction exercée et les exigences pour le capteur en termes de résistance mécanique sont moindres. Le choix parmi les capteurs disponibles sur le marché s'en trouve élargi et les capteurs de déformation adaptés sont généralement moins onéreux que les capteurs de force utilisés dans l'art antérieur.

Le dispositif proposé permet, au cours de l'opération d'insertion de l'implant chirurgical, de renseigner en temps réel le praticien sur le niveau de contact atteint entre l'implant et l'os récepteur. Outre son coût réduit, ce dispositif a l'avantage d'être simple d'utilisation. En particulier, avec ce dispositif, le geste du praticien lors de l'opération peut rester le même, auquel cas Le praticien n'a pas à apprendre de nouveaux gestes et peut tirer profit de l'expérience qu'il a déjà acquise avec les dispositifs conventionnels.

Outre les caractéristiques qui viennent d'être mentionnées plus haut, le dispositif proposé peut présenter une ou plusieurs des caractéristiques suivantes, considérées isolément ou selon des combinaisons techniquement possibles:

- l'unité de traitement comprend un filtre passe-bas permettant d'atténuer dans le signal de mesure les fréquences supérieures à une valeur seuil comprise entre 20% et 100% de la fréquence de résonnance de l'impacteur,
- l'unité de traitement comprend un filtre passe-bas permettant d'atténuer dans le signal de mesure les fréquences supérieures à une valeur seuil comprise entre 1 kHz et 35 kHz, en particulier entre 5 kHz et 20 kHz.
- l'impacteur a une face de frappe adaptée pour impacter la surface d'impact, une face opposée à la face de frappe et des faces latérales s'étendant entre la face de frappe et la face opposée, et ledit au moins un capteur est fixé sur au moins une des faces latérales ou sur la face opposée,
- l'impacteur a une face avant sur laquelle est formée une protubérance, la face avant de cette protubérance formant la face de frappe, et le capteur est disposé sur une face latérale de la protubérance.

Dans certains modes de réalisation, l'indicateur proposé correspond à la moyenne de la déformation, calculée sur une fenêtre temporelle de durée programmable positionnée sur ledit signal de mesure.

Le début de ladite fenêtre temporelle peut coïncider avec l'instant où le signal de mesure atteint son amplitude maximale. La durée de ladite fenêtre temporelle peut alors être comprise entre 0,1 ms et 5 ms, avantageusement entre 0,1 ms et 0,5 ms et avantageusement encore égale à 0,25 ms. En variante, le début de la fenêtre temporelle peut être défini avec un retard prédéterminé, pouvant varier de 0 à 5 ms, par rapport à l'instant où le signal de mesure atteint son amplitude maximale.

Par exemple, l'indicateur IN1 est calculé de la façon suivante :

$$IN1 = \frac{1}{A_1 \cdot (t_2 - t_1)} \int_{t_1}^{t_2} A(t).dt$$

où :

A(t) correspond à l'amplitude dudit signal de mesure à l'instant t ;
$A_1$ correspond à l'amplitude maximale dudit signal de mesure ; et
$t_1$ et $t_2$ correspondent respectivement aux instants de début et de fin de la fenêtre temporelle.

En variante, ledit indicateur est déterminé par lesdits moyens de traitement comme étant la durée d'impact mesurée à partir du signal de mesure (i.e. de la variation temporelle de la déformation lors d'un impact). Cette durée correspond à la durée (ti2 - ti1) séparant un instant ti1 correspondant au début de l'impact et un instant ti2 correspondant à la fin de l'impact. Par exemple, l'instant ti2 de fin d'impact peut être choisi comme l'instant où l'amplitude de la déformation devient inférieure à une valeur limite prédéfinie.

Dans d'autres modes de réalisation, l'indicateur correspond à la durée d'une fenêtre temporelle, le début de cette fenêtre temporelle étant défini par rapport à un instant correspondant au premier pic d'amplitude maximale du signal de mesure et la fin de cette fenêtre temporelle étant définie par rapport à un instant correspondant au deuxième pic d'amplitude maximale du signal de mesure.

Il a été montré que tous ces indicateurs étaient représentatifs du niveau de contact entre l'implant et l'os récepteur et constituaient des indicateurs fiables.

En règle générale, au fur et à mesure des impacts, le niveau du contact entre l'implant et l'os-récepteur augmente d'abord et tend à se stabiliser ensuite.

Lorsque le niveau du contact implant-os devient stable, il est généralement considéré comme suffisant. L'indicateur proposé, représentatif du niveau de contact, adopte un comportement similaire et tend, au fur et à mesure des impacts, à augmenter/diminuer d'abord et à se stabiliser ensuite autour d'une valeur stationnaire supérieur/inférieure à une valeur seuil. Les valeurs des exemples d'indicateurs précités diminuent ainsi au fur et à mesure des impacts, jusqu'à atteindre un palier sensiblement stable.

Il est donc possible de dériver du comportement de l'indicateur une condition pour émettre le signal d'alerte (e.g. une lumière, un son, une vibration, etc.). Le praticien, averti par ce signal, sait alors qu'il doit arrêter d'impacter l'implant, le niveau de contact entre l'implant et l'os étant considéré comme optimal ou, en tous cas, comme suffisant. Ainsi, dans certains modes de réalisation, le dispositif comprend un système d'alerte relié à l'unité de traitement et coopérant avec celle-ci de manière à émettre un signal d'alerte lorsque l'indicateur converge vers une valeur stationnaire lors d'impacts successifs, ou lorsque l'indicateur dépasse une valeur seuil prédéterminée. Cette valeur seuil peut être déterminée expérimentalement au cours de tests, ou être déterminée par calcul. Par exemple, on peut réaliser des tests sur des cadavres et choisir, comme valeur seuil, la valeur de l'indicateur à partir de laquelle on constate un niveau de contact suffisant entre l'implant et l'os récepteur.

Dans certains modes de réalisation, l'impacteur est un marteau, ou équivalent, et comprend un manche surmonté d'une tête de frappe. En particulier, l'impacteur peut avoir sensiblement la même forme et le même poids que les impacteurs couramment utilisés jusqu'à présent. Ainsi, les praticiens expérimentés sont immédiatement en mesure de manier correctement l'impacteur.

On notera que la surface d'impact peut-être couplée directement à l'implant en ce sens qu'il peut s'agir d'une des surfaces de l'implant, ou peut être indirectement couplée à l'implant en ce sens qu'il peut s'agir d'une surface d'un instrument, ou ancillaire de pose, venant lui-même au contact de l'implant. Dans ce dernier cas, l'impacteur exerce la force d'impaction sur l'implant par l'intermédiaire de l'ancillaire de pose. En d'autres termes, la force d'impaction est exercée sur l'ancillaire de pose et transmise par celui-ci à l'implant.

Dans certains modes de réalisation, le dispositif comprend un ancillaire de pose ayant une extrémité arrière formant ladite surface d'impact et une extrémité avant adaptée pour coopérer avec l'implant, l'impacteur exerçant la force d'impaction sur l'implant par l'intermédiaire de l'ancillaire de pose.

L'extrémité avant de l'ancillaire de pose peut coopérer avec l'implant par simple contact. En variante, l'extrémité avant de l'ancillaire de pose peut être attachée mécaniquement à l'implant orthopédique de manière amovible, par exemple par vissage. Le fait d'attacher l'ancillaire à l'implant permet, généralement, d'obtenir un meilleur signal de mesure. L'ancillaire est amovible de manière à pouvoir détacher facilement l'ancillaire de l'implant une fois ce dernier en position.

Le présent exposé concerne également un ensemble comprenant un dispositif tel que précédemment décrit et un implant chirurgical, en particulier une tige fémorale ou une cupule cotyloïdienne.

Un procédé pour insérer en force un implant chirurgical dans un os récepteur, par impaction, est également décrit ici, bien que n'entrant pas dans l'étendue de l'invention telle que revendiquée. Dans ce procédé:

- on fournit un dispositif tel que précédemment décrit,
- on exerce avec l'impacteur une force d'impaction sur l'implant, en impactant une surface d'impact couplée à l'implant, de manière à insérer l'implant,
- on calcule l'indicateur pour avoir une idée du niveau de contact entre l'implant et l'os récepteur.

Dans certains modes de mise en œuvre, l'indicateur peut être calculé une ou plusieurs fois, en fin d'insertion de l'implant, afin de valider la bonne implantation de l'implant.

En variante, l'indicateur peut être calculé lors d'impacts successifs et on arrête d'impacter la surface d'impact lorsque l'indicateur converge vers une valeur stationnaire lors d'impacts successif, ou lorsque l'indicateur dépasse une valeur seuil prédéterminée.

L'implant chirurgical peut être, mais n'est pas nécessairement, une cupule cotyloïdienne. Dans ce cas, on impacte la cupule avec l'impacteur de manière à insérer en force la cupule dans une cavité de l'os iliaque. L'indicateur peut être calculé en fin d'insertion afin de valider la pose de la cupule.

Les avantages d'un tel procédé découlent des avantages du dispositif utilisé.

Les caractéristiques et avantages précités, ainsi que d'autres, apparaîtront à la lecture de la description détaillée qui suit, d'exemples de réalisation du dispositif proposé. Cette description détaillée fait référence aux dessins annexés.

BREVE DESCRIPTION DES DESSINS

Les dessins annexés sont schématiques et ne sont pas à l'échelle, ils visent avant tout à illustrer les principes de l'invention.

- la figure 1 est une représentation schématique d'un dispositif pour insérer un implant chirurgical, comprenant un impacteur muni d'un capteur de déformation,
- la figure 2 est un graphique représentant des exemples de signaux obtenus à l'aide du capteur de déformation de la figure 1, et
- la figure 3 représente schématiquement un autre exemple d'impacteur.

La figure (FIG) 1 représente un exemple de dispositif 1 pour insérer en force un implant chirurgical dans un os récepteur, par impaction. Dans cet exemple, l'implant est un implant pour prothèse, en particulier pour prothèse de hanche. La grande majorité des prothèses de hanche ont en commun une première partie fixée dans le fémur et une deuxième partie fixée dans le bassin. La première partie comprend une tige fémorale destinée à être insérée en force dans le canal médullaire du fémur et une tête prothétique formée d'une pièce sphérique, montée sur la tige fémorale et se substituant au col du fémur. La seconde partie comprend un cotyle prothétique destiné à être inséré dans la cavité cotyloïdienne située sur la face latérale de l'os iliaque du bassin pour remplacer la partie articulaire du bassin. Le cotyle prothétique peut comprendre une cupule cotyloïdienne, qui est une pièce approximativement hémisphérique, généralement en métal, insérée dans la cavité cotyloïdienne et dans laquelle se place un insert avec lequel s'articule la tête prothétique.

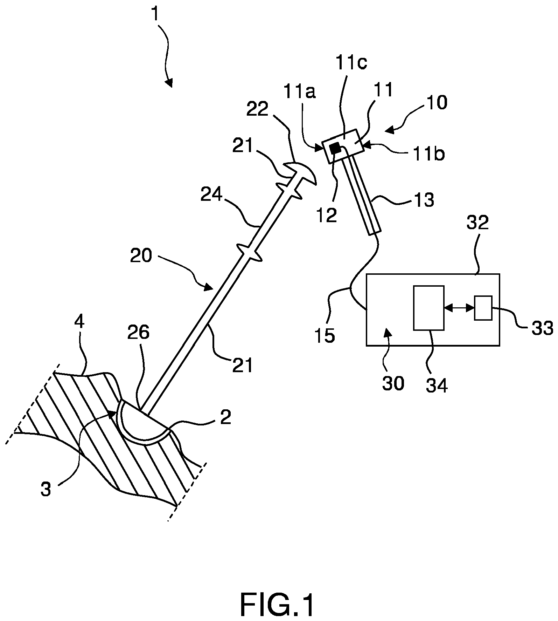

L'implant de la figure 1 est une cupule cotyloïdienne 2. Cette cupule 2 est destinée à être insérée progressivement par impaction dans la cavité cotyloïdienne 3 de l'os iliaque 4 du bassin d'un patient, la cavité 3 ayant été préalablement préparée par le praticien pour recevoir la cupule 2. L'insertion de la cupule 2 dans la cavité 3 est réalisée par impaction au moyen d'un impacteur 10, typiquement un marteau. Un ancillaire de pose 20 peut également être utilisé. Dans ce cas, le praticien frappe l'ancillaire 20 avec l'impacteur 10 et l'ancillaire 20 transmet la force d'impaction à la cupule 2. Au fur et à mesure des impacts, la cupule 2 s'enfonce dans la cavité 3.

L'ancillaire de pose 20 est fait d'une tige rigide 21 à l'extrémité arrière de laquelle est fixé rigidement un pommeau ayant une surface bombée, formant une surface d'impact 22. En parcourant la tige 21 depuis son extrémité arrière jusqu'à son extrémité avant, la tige 21 comprend ledit pommeau, un tronçon formant une poignée 24, un tronçon avant et une tête 26 de préhension de la cupule 2. L'avant et l'arrière sont définis ici par rapport à la direction d'avancement de l'ancillaire 20 lors de l'impaction.

Le dispositif 1 comprend également un outil de percussion ou impacteur 10, de type marteau ou équivalent, comprenant un manche 13 surmonté d'une tête de frappe 11. La tête 11 a une face de frappe 11a adaptée pour impacter la surface d'impact 22 de l'ancillaire 20, une face 11b opposée à la face de frappe 11a et des faces latérales 11c s'étendant entre la face de frappe 11a et la face opposée 11b. Lorsqu'il souhaite enfoncer la cupule 2 dans l'os 4, le praticien saisit l'ancillaire de pose 20 d'une main, par la poignée 24, et le manche 13 de l'impacteur 10 de l'autre main. Il frappe ensuite la surface d'impact 22 de l'ancillaire 20 avec la face de frappe 11a de l'impacteur 10. La force d'impaction générée par l'impacteur 10 est transmise à la cupule 2 par l'intermédiaire de l'ancillaire 20.

Selon l'invention, l'impacteur 10 est équipé d'un ou plusieurs capteurs de déformation 12. Ce ou ces capteurs 12 sont destinés à détecter la déformation de l'impacteur 10, plus précisément de la tête de frappe 11, lors de chaque impact et à convertir cette déformation en un signal électrique exploitable.

Dans l'exemple de la figure 1, l'impacteur 10 est équipé d'un capteur de déformation 12 positionné sur une des faces latérales 11c de la tête de frappe 11. En l'occurrence, le capteur 12 est positionné sur la face latérale 11c s'étendant sensiblement parallèlement à la direction de l'axe du manche 13. Plus précisément, vu de côté (comme sur la FIG 1), le capteur 12 est fixé sur la partie avant de la face latérale 11c, entre la face de frappe 11a et l'axe du manche 13. Cette disposition particulière du capteur 12 sur la tête de frappe 11 permet d'optimiser les déformations mesurées et, donc, d'obtenir un signal de mesure porteur de meilleures informations et plus facilement exploitable. Cependant, de manière générale, le ou les capteurs 12 de déformation peuvent être positionnés sur n'importe quelle face de l'impacteur 10 en dehors de la face de frappe 11a (i.e. sur les faces latérales 11c ou sur la face opposée 11b).

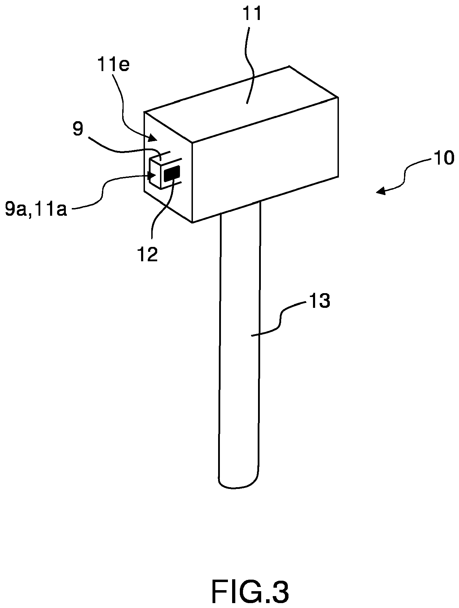

En variante, comme représenté sur la FIG 3, l'impacteur 10 a une face avant 11e sur laquelle est formée une protubérance 9. La face avant 9a de cette protubérance 9 forme la face de frappe 11a de l'impacteur 10, et le capteur 12 est fixé sur une face latérale de la protubérance 9, i.e. sur une face de la protubérance 9 s'étendant entre la face de frappe 11a et la face avant 11e de l'impacteur. Cette disposition particulière du capteur 12 sur la tête de frappe 11 de l'impacteur 10 permet d'optimiser les déformations mesurées et de s'affranchir, au moins en partie, des fréquences de résonance propres à l'impacteur 10. Elle permet ainsi d'obtenir un signal de mesure porteur de meilleures informations et plus facilement exploitable.

Le capteur de déformation 12 est fixé sur la tête de frappe 11, par exemple par collage ou tout autre moyen de fixation approprié, de sorte que la déformation de la tête de frappe 11 provoque la déformation du capteur 12. Le capteur 12 est, par exemple, un capteur à jauge comprenant un élément de mesure élastique dont la déformation est d'abord convertie en une variation de résistance électrique de la jauge, pour générer ensuite un signal de sortie électrique. En variante, il peut s'agir d'un capteur piézo-électrique reposant sur les propriétés piézo-électriques d'un matériau (e.g. du quartz ou des céramiques synthétiques) qui génère une charge électrique lorsqu'il se déforme. Le capteur 12 est fixé sur la tête de frappe 11 de l'impacteur 10, par exemple par collage ou tout autre moyen de fixation approprié, de sorte que la déformation de la tête de frappe 11 provoque la déformation du capteur. A titre d'exemple, le capteur de déformation 12 peut être un capteur mesurant 20x20 mm, commercialisé sous la marque PI et la référence PIC255, et être collé sur l'impacteur 10 avec de la colle époxy susceptible de résister à des températures obtenues dans un autoclave.

Le dispositif comprend également une unité de traitement 30 couplée au capteur 12 et configurée pour évaluer le degré d'insertion de la cupule 2 dans l'os récepteur 4, à partir des signaux de mesure délivrés par le capteur 12. Cette unité de traitement 30 comprend, par exemple, un microcontrôleur 34. L'unité de traitement 30 peut être logée dans un boîtier externe 32. En variante, l'unité de traitement 30 peut être intégrée à l'impacteur 10. Selon une autre variante (non représentée), l'unité de traitement 30 peut être formée d'éléments séparés comme un micro-ordinateur relié à un module d'acquisition de données lui-même relié au capteur 12.

La liaison entre le capteur 12 et l'unité de traitement 30 est, dans l'exemple de la FIG 1, réalisée de manière filaire au moyen d'un câble 15. En variante, la transmission des signaux de mesure fournis par le capteur 12 peut se faire au moyen d'une liaison sans fil, auquel cas le capteur 12 est équipé d'une antenne ou équivalent.

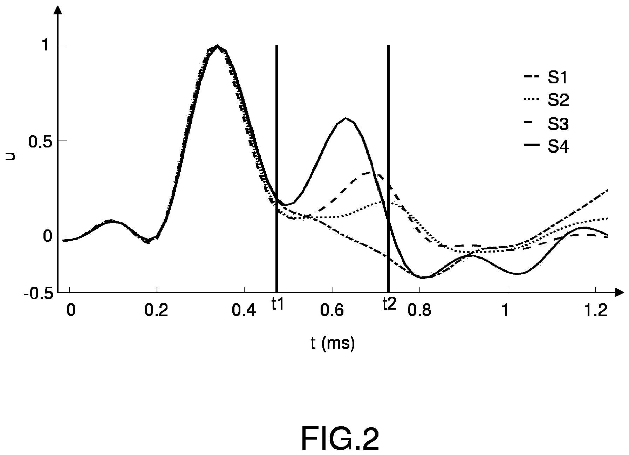

Lors de chaque impact effectué par le praticien sur la cupule 3 au moyen de l'impacteur 10, via l'ancillaire de pose 20, le capteur 12 mesure la déformation de la tête de frappe 11 de l'impacteur 10 et fournit un signal de mesure représentant la variation temporelle de cette déformation pendant l'impact. On considère que l'impact commence à partir de l'instant où l'impacteur 10 et l'implant entrent en contact, directement ou indirectement (i.e. via l'ancillaire 20), et dure pendant un certain laps de temps après cet instant. En tout état de cause, ce laps de temps est inférieur à 50 ms. Des exemples de signaux fournis par le capteur 12 sont représentés sur la FIG. 2 et décrits ci-après.

Les inventeurs ont eu l'idée de s'intéresser à un tel signal de mesure et ont mis en évidence le fait que ce signal portait des informations sur le niveau de contact entre la cupule 2 et l'os 4. En particulier, les inventeurs ont réussi à déterminer, à partir du signal de mesure recueilli, un indicateur représentatif du niveau de contact entre l'os 4 et la cupule 2, comme expliqué ci-après.

Pour essayer d'expliquer le lien entre le signal de mesure recueilli et le niveau de contact os-implant, l'explication suivante peut être avancée. L'impacteur 10 exerce sur la cupule 2, via l'ancillaire 20, une force d'impaction qui est à l'origine de modes de vibration dans l'ensemble du système formé par l'impacteur 10, l'ancillaire 20, la cupule 2 et l'os 4 lorsque ces éléments sont tous en contact lors de l'impact. Ces modes de vibration dépendent essentiellement des modes de vibration du système os-implant (i.e. du système os-cupule) qui dépendent à leur tour du niveau de contact entre l'implant et l'os. Schématiquement, plus le niveau de contact os-implant est élevé, plus le système os-implant est rigide et plus les fréquences de résonance des modes de vibration sont élevées.

Dans certains modes de réalisation, l'unité de traitement 30 comprend un filtre passe-bas permettant d'atténuer dans le signal de mesure les fréquences supérieures à une valeur seuil. Cette valeur seuil est notamment choisie pour atténuer dans le signal l'influence de la résonnance de l'impacteur lui-même. La fréquence de résonnance de l'impacteur est constante pour un impacteur donné et nettement plus élevée que les fréquences des signaux porteurs d'information sur le système os-implant. En particulier, pour l'insertion de la cupule 2 de la FIG. 1, l'impacteur 10 peut avoir une fréquence de résonnance d'environ 20 kHz ou plus, tandis que les fréquences du signal utile fourni par le capteur 12 sont généralement inférieures à 5 kHz. La valeur seuil peut ainsi être choisie entre 5 kHz et 20 kHz.

Le filtre passe-bas permet donc d'atténuer dans le signal de mesure les fréquences proches et supérieures à la fréquence de résonnance de l'impacteur 10, qui sont caractéristiques des modes de vibration propres à l'impacteur 10 et qui ne portent pas d'information sur le niveau de contact entre l'implant et l'os, tout en conservant les fréquences plus basses, qui sont caractéristiques des modes de vibration de l'ensemble du système formé par l'impacteur 10, l'ancillaire 20, la cupule 2 et l'os 4, et qui portent une information sur le niveau de contact os-cupule. La qualité du signal de mesure en termes d'informations utiles s'en trouve améliorée et le signal est plus facilement exploitable.

La FIG 2 est un graphique représentant des exemples de signaux obtenus à l'aide du capteur de déformation 12 pour différents niveaux de contact os-implant. Ces signaux ont été obtenus lors de quatre essais visant à mettre en évidence la corrélation entre l'indicateur proposé et le niveau de contact entre l'implant et l'os. Ces quatre essais ont été réalisés en utilisant quatre échantillons d'os bovins obtenus dans une boucherie. Pour chaque échantillon, une cavité a été réalisée dans les mêmes conditions et avec le même matériel que lors d'une opération chirurgicale. La cupule 2 a ensuite été positionnée au dessus de la cavité et des efforts d'impaction ont été appliqués comme cela est fait en clinique. Pour chaque échantillon, la cupule 2 a été enfoncée jusqu'à ce qu'elle soit totalement enfoncée dans l'os. Une fois la cupule totalement enfoncée, la cupule 2 a été modérément frappée avec l'impacteur 10 de la FIG 1. Cet impact modéré n'avait pas pour but de modifier la position de la cupule 2 dans l'os, mais de calculer l'indicateur à partir du signal de mesure émis par capteur de déformation 12. Une fois l'indicateur calculé, la cupule 2 a été arrachée de l'os en exerçant sur celle-ci une force de traction suivant l'axe de la cupule 2. Cette force de traction, ou force d'arrachement, reflète le niveau de contact entre l'implant et l'os récepteur: plus le niveau de contact os-implant est élevé, plus il est difficile d'arracher l'implant de l'os, et plus la force d'arrachement est élevée.

Sur le graphique de la FIG 2, le temps t en millisecondes (ms) est reporté en abscisse, et la tension u normalisée du signal électrique fourni par le capteur 12 lors des quatre essais précités est reportée en ordonnée. Cette tension est directement proportionnelle à la déformation de la tête de frappe 11 mesurée par le capteur 12. Les quatre signaux sont référencés S1 à S4. Ces signaux ont été normalisés et filtrés.

Pour chaque signal, l'indicateur IN1 a été calculé de la façon suivante :

$$IN1 = \frac{1}{A_1.(t_2 - t_1)} \int_{t_1}^{t_2} A(t).dt$$

où :

A(t) correspond à l'amplitude du signal de mesure à l'instant t;

$A_1$ correspond à l'amplitude maximale dudit signal de mesure; et

$t_1$ et $t_2$ correspondent respectivement aux instants de début et de fin d'une fenêtre temporelle de durée programmable positionnée sur ledit signal de mesure.

En l'occurrence, dans cet exemple, les instants $t_1$ et $t_2$ sont repérés sur la FIG 2 et ont été déterminés par la mise en œuvre d'un processus d'optimisation.

Le tableau ci-dessous donne, pour chaque signal S1 à S4 les valeurs de l'indicateur calculé et de la force d'arrachement, en Newton, mesurée.

|    | Indicateur IN1 | Force d'arrachement (N) |
|----|----------------|-------------------------|
| S1 | 0,1            | 13,6                    |
| S2 | 0,26           | 50,8                    |
| S3 | 0,48           | 95,4                    |
| S4 | 0.94           | 179                     |

Ces résultats illustrent la corrélation qui existe entre l'indicateur proposé et la force d'arrachement, et donc entre l'indicateur proposé et le niveau de contact os-implant. Cet indicateur peut, par conséquent, être utilisé pour évaluer le niveau de contact os-implant.

En référence à la FIG 1, le dispositif 10 peut comprendre un système d'alerte 33 pour émettre un signal d'alerte (par exemple, un signal sonore, visuel et/ou tactile). Le système d'alerte 33 est relié à l'unité de traitement 30 et coopère avec celle-ci pour alerter le praticien lorsque le niveau de contact entre la cupule 2 et l'os est jugé suffisant sur la base de l'indicateur IN1, c'est-à-dire, par exemple, dès que l'indicateur IN1 descend en dessous de la valeur seuil S1 ou lorsque cet indicateur IN1 tend à se stabiliser. Dès lors, le practicien dispose en temps réel d'une information fiable lui indiquant qu'il a atteint un niveau de contact os-implant suffisant. Il en conclut qu'il peut arrêter d'impacter la cupule 2, ce qui diminue le risque d'endommager l'os 4, en particulier d'induire une fracture ou des microfissures dans l'os.

L'exemple qui vient d'être décrit, concernant l'insertion d'une cupule 2 dans un os iliaque 4, est donné à titre illustratif et non limitatif, une personne du métier pouvant facilement tirer profit de l'indicateur proposé par les inventeurs avec d'autres types d'implants, sans sortir du cadre de l'invention. Autrement dit, la cupule 2 et l'os 4 ne sont, respectivement, que des exemples d'implant chirurgical et d'os récepteur au sens de l'invention.

En particulier, le dispositif proposé peut être utilisé pour des implants pour prothèse de hanche autres qu'une cupule cotyloïdienne (e.g. pour une tige fémorale), des implants pour prothèse de genou, d'épaule, de rachis, de cheville, etc. et, plus généralement, tout type d'implant chirurgical nécessitant d'être inséré en force par impaction dans un os récepteur. Il peut également être utilisé pour l'insertion d'instruments chirurgicaux insérés temporairement dans le corps d'un patient et, par exemple, pour l'insertion d'une râpe chirurgicale comme une râpe fémorale pour prothèse de hanche. Les râpes fémorales sont prévues pour être insérées en force par impaction dans le canal médullaire afin de préparer ce canal à recevoir la tige fémorale. Ces râpes sont impactées directement par un impacteur, avec ou sans l'intermédiaire d'un ancillaire de pose. Si aucun ancillaire de pose n'est utilisé, la surface d'impact est alors constituée par une surface située au niveau de l'extrémité arrière d'une partie de préhension de la râpe.

Enfin, les différentes caractéristiques des modes ou exemples de réalisation décrits dans le présent exposé peuvent être considérées isolément ou être combinées entre elles. Lorsqu'elles sont combinées, ces caractéristiques peuvent l'être comme décrit ci-dessus ou différemment, l'invention ne se limitant pas aux combinaisons spécifiques précédemment décrites. En particulier, sauf précision contraire ou incompatibilité technique, une caractéristique décrite en relation avec un mode ou exemple de réalisation peut être appliquée de manière analogue à un autre mode ou exemple de réalisation.

## Revendications

1. Dispositif pour insérer en force un implant chirurgical dans un os récepteur (4), par impaction, comprenant:

un outil de percussion, ou impacteur (10), adapté pour impacter une surface d'impact (22) couplée à l'implant et exercer une force d'impaction sur l'implant,
au moins un capteur (12) associé à l'impacteur (10), et
une unité de traitement (30) reliée au capteur (12),
**caractérisé en ce que**:

le capteur (12) est apte à mesurer la déformation de l'impacteur (10), et à fournir un signal de mesure représentant la variation temporelle de cette déformation lors d'un impact, et

l'unité de traitement (30) est configurée pour calculer, à partir de la variation temporelle de la déformation de l'impacteur (10) lors de l'impact, un indicateur représentatif du niveau de contact entre l'implant et l'os récepteur (4).

**2.** Dispositif selon la revendication 1, dans lequel l'unité de traitement (30) comprend un filtre passe-bas permettant d'atténuer dans le signal de mesure les fréquences supérieures à une valeur seuil comprise entre 20% et 100% de la fréquence de résonance de l'impacteur.

**3.** Dispositif selon la revendication 1 ou 2, dans lequel l'unité de traitement (30) comprend un filtre passe-bas permettant d'atténuer dans le signal de mesure les fréquences supérieures à une valeur seuil comprise entre 1 kHz et 35 kHz, en particulier entre 5 kHz et 20 kHz.

**4.** Dispositif selon l'une quelconque des revendications 1 à 3, dans lequel l'impacteur (10) a une face de frappe (11a) adaptée pour impacter la surface d'impact (22), une face opposée (11b) à la face de frappe et des faces latérales (11c) s'étendant entre la face de frappe (11a) et la face opposée (11b), et dans lequel ledit au moins un capteur (12) est fixé sur au moins une des faces latérales (11c) ou sur la face opposée.

**5.** Dispositif selon l'une quelconque des revendications 1 à 4, dans lequel l'impacteur (10) a une face avant (11e) sur laquelle est formée une protubérance (9), la face avant (9a) de la protubérance (9) formant la face de frappe (11a), et dans lequel le capteur (12) est disposé sur une face latérale de la protubérance (9).

**6.** Dispositif selon l'une quelconque des revendications 1 à 5, dans lequel l'indicateur (IN1) correspond à la moyenne de la déformation, calculée sur une fenêtre temporelle de durée programmable positionnée sur ledit signal de mesure.

**7.** Dispositif selon la revendication 6, dans lequel l'indicateur (IN1) est calculé de la façon suivante :

$$IN1 = \frac{1}{A_1.(t_2 - t_1)} \int_{t_1}^{t_2} A(t).dt$$

où:

A(t) correspond à l'amplitude dudit signal de mesure à l'instant t ;

A₁ correspond à l'amplitude maximale dudit signal de mesure ; et

t₁ et t₂ correspondent respectivement aux instants de début et de fin de ladite fenêtre temporelle.

**8.** Dispositif selon l'une quelconque des revendications 1 à 7, dans lequel l'indicateur correspond à la durée d'une fenêtre temporelle, le début de cette fenêtre temporelle étant défini par rapport à un instant correspondant au premier pic d'amplitude maximale du signal de mesure et la fin de cette fenêtre temporelle étant définie par rapport à un instant correspondant au deuxième pic d'amplitude maximale du signal de mesure.

**9.** Dispositif selon l'une quelconque des revendications 1 à 8 comprenant, en outre, un système d'alerte (33) relié à l'unité de traitement (30) et coopérant avec celle-ci de manière à émettre un signal d'alerte lorsque l'indicateur converge vers une valeur stationnaire lors d'impacts successifs, ou lorsque l'indicateur dépasse une valeur seuil prédéterminée.

**10.** Dispositif selon l'une quelconque des revendications 1 à 9 comprenant, en outre, un ancillaire de pose (20) ayant une extrémité arrière formant ladite surface d'impact et une extrémité avant adaptée pour coopérer avec l'implant, l'impacteur (10) exerçant la force d'impaction sur l'implant par l'intermédiaire de l'ancillaire de pose (20).

**11.** Dispositif selon l'une quelconque des revendications 1 à 10, dans lequel l'impacteur (10) est un marteau, ou équivalent, et comprend un manche (13) surmonté d'une tête de frappe (11), et dans lequel le capteur est fixé sur la tête de frappe (11) de sorte que la déformation de la tête de frappe (11) provoque la déformation du capteur (12).

**12.** Dispositif selon la revendication 11, dans lequel la tête de frappe (11) a une face de frappe (11a) adaptée pour impacter la surface d'impact (22), une face opposée (11b) à la face de frappe (11a) et des faces latérales (11c) s'étendant entre la face de frappe (11a) et la face opposée (11b), et dans lequel le capteur (12) est positionné sur une des faces latérales (11c) s'étendant sensiblement parallèlement à la direction de l'axe du manche (13) et, plus précisément, sur la partie avant de cette face latérale (11c), entre la face de frappe (11a) et l'axe du manche (13).

**13.** Ensemble comprenant un dispositif selon l'une quelconque des revendications 1 à 12 et un implant chirurgical, en particulier une tige fémorale ou une cupule cotyloïdienne (2).

**Patentansprüche**

1. Vorrichtung zum Eintreiben eines chirurgischen Implantats in einen Empfängerknochen (4) durch Einpressen, aufweisend:

   ein Schlagwerkzeug oder Einschläger (10), das dazu geeignet ist, auf eine mit dem Implantat gekoppelte Aufprallfläche (22) zu schlagen und eine Schlagkraft auf das Implantat auszuüben, mindestens einen Sensor (12), der mit dem Einschläger (10) verbunden ist, und eine Behandlungseinheit (30), die mit dem Sensor (12) verbunden ist,
   **dadurch gekennzeichnet, dass**
   der Sensor (12) geeignet ist, die Verformung des Einschlägers (10) zu messen und ein Messsignal zu liefern, das die zeitliche Veränderung dieser Verformung bei einem Aufprall darstellt, und die Behandlungseinheit (30) so konfiguriert ist, dass sie aus der zeitlichen Variation der Verformung des Einschlägers (10) beim Aufprall einen Indikator berechnet, der für das Kontaktniveau zwischen dem Implantat und dem Empfängerknochen (4) repräsentativ ist.

2. Vorrichtung nach Anspruch 1, bei der die Behandlungseinheit (30) einen Tiefpassfilter aufweist, der es ermöglicht, im Messsignal die höheren Frequenzen zu dämpfen, die über einem Schwellenwert liegen, der zwischen 20 % und 100 % der Resonanzfrequenz des Einschlägers liegt.

3. Vorrichtung nach Anspruch 1 oder 2, bei der die Behandlungseinheit (30) einen Tiefpassfilter aufweist, der es ermöglicht, im Messsignal die höheren Frequenzen zu dämpfen, die über einem Schwellenwert zwischen 1 kHz und 35 kHz, insbesondere zwischen 5 kHz und 20 kHz, liegen.

4. Vorrichtung nach einem der Ansprüche 1 bis 3, wobei der Einschläger (10) eine Schlagfläche (11a), die zum Aufprall auf die Aufprallfläche (22) geeignet ist, eine der Schlagfläche gegenüberliegende Fläche (11b) und Seitenflächen (11c), die sich zwischen der Schlagfläche (11a) und der gegenüberliegenden Fläche (11b) erstrecken, aufweist, und wobei der mindestens eine Sensor (12) an mindestens einer der Seitenflächen (11c) oder an der gegenüberliegenden Fläche befestigt ist.

5. Vorrichtung nach einem der Ansprüche 1 bis 4, wobei der Einschläger (10) eine Vorderseite (11e) aufweist, auf der ein Vorsprung (9) ausgebildet ist, wobei die Vorderseite (9a) des Vorsprungs (9) die Schlagfläche (11a) bildet, und wobei der Sensor (12) an einer Seitenfläche des Vorsprungs (9) angeordnet ist.

6. Vorrichtung nach einem der Ansprüche 1 bis 5, wobei der Indikator (IN1) dem Mittelwert der Verformung entspricht, der über ein Zeitfenster mit programmierbarer Dauer berechnet wird, in dem das Messsignal aufgenommen wurde.

7. Vorrichtung nach Anspruch 6, wobei der Indikator (IN1) wie folgt berechnet wird:

$$IN1 = \frac{1}{A_1 \cdot (t_2 - t_1)} \int\limits_{t_1}^{t_2} A(t).dt$$

wobei:

   A(t) der Amplitude des genannten Messsignals zum Zeitpunkt t entspricht;
   $A_1$ der maximalen Amplitude des Messsignals entspricht; und
   $t_1$ und $t_2$ dem Start- bzw. Endzeitpunkt des Zeitfensters entsprechen.

8. Vorrichtung nach einem der Ansprüche 1 bis 7, bei der der Indikator der Dauer eines Zeitfensters entspricht, wobei der Beginn dieses Zeitfensters in Bezug auf einen Zeitpunkt definiert ist, der dem ersten Spitzenwert der maximalen Amplitude des Messsignals entspricht, und das Ende dieses Zeitfensters in Bezug auf einen Zeitpunkt definiert ist, der dem zweiten Spitzenwert der maximalen Amplitude des Messsignals entspricht.

9. Vorrichtung nach einem der Ansprüche 1 bis 8, die außerdem ein Warnsystem (33) aufweist, das mit der Behandlungseinheit (30) verbunden ist und mit dieser so zusammenarbeitet, dass es ein Warnsignal ausgibt, wenn der Indikator bei aufeinanderfolgenden Stößen gegen einen stationären Wert konvergiert oder wenn der Indikator einen vorgegebenen Schwellenwert überschreitet.

10. Vorrichtung nach einem der Ansprüche 1 bis 9, die außerdem eine Einsetzhilfe (20) aufweisend ein hinteres Ende, das die Aufprallfläche bildet, und ein vorderes Ende, das so beschaffen ist, dass es mit dem Implantat zusammenwirkt, aufweist, wobei der Einschläger (10) die Schlagkraft über die Einsetzhilfe (20) auf das Implantat ausübt.

11. Vorrichtung nach einem der Ansprüche 1 bis 10, wobei der Einschläger (10) ein Hammer oder dergleichen ist und einen Stiel (13) aufweist, auf dem ein Schlagkopf (11) sitzt, und wobei der Sensor am Schlagkopf (11) befestigt ist, so dass die Verformung des Schlagkopfes (11) die Verformung des Sensors (12) bewirkt.

**12.** Vorrichtung nach Anspruch 11, wobei der Schlagkopf (11) eine Schlagfläche (11a), die zum Auftreffen auf die Aufprallfläche (22) geeignet ist, eine der Schlagfläche (11a) gegenüberliegende Fläche (11b) und Seitenflächen (11c), die sich zwischen der Schlagfläche (11a) und der gegenüberliegenden Fläche (11b) erstrecken, aufweist, und wobei der Sensor (12) auf einer der Seitenflächen (11c), die sich im Wesentlichen parallel zur Richtung der Achse des Griffs (13) erstrecken, und genauer gesagt auf dem vorderen Teil dieser Seitenfläche (11c) zwischen der Schlagfläche (11a) und der Achse des Griffs (13) positioniert ist.

**13.** Set aufweisend eine Vorrichtung nach einem der Ansprüche 1 bis 12 und ein chirurgisches Implantat, insbesondere einen Oberschenkelschaft oder einer Hüftgelenkspfanne (2).

**Claims**

**1.** A device for forcibly inserting a surgical implant into a receiving bone (4), by impaction, comprising:

a percussion tool, or impactor (10), for impacting an impact surface (22) coupled to the implant and exerting an impact force on the implant,
at least one sensor (12) associated with the impactor (10), and
a processing unit (30) connected to the sensor (12),
**characterized in that**:

the sensor (12) is adapted for measuring the deformation of the impactor (10), and for providing a measurement signal representing the temporal variation of this deformation during an impact, and
the processing unit (30) is configured to calculate, on the basis of the temporal variation of the deformation of the impactor (10) during the impact, an indicator representative of the level of contact between the implant and the receiving bone (4).

**2.** The device according to claim 1, wherein the processing unit (30) comprises a low-pass filter for attenuating in the measurement signal the frequencies above a threshold value of between 20% and 100% of the resonance frequency of the impactor.

**3.** The device according to claim 1 or 2, wherein the processing unit (30) comprises a low-pass filter for attenuating in the measurement signal the frequencies above a threshold value of between 1 kHz and 35 kHz, in particular between 5 kHz and 20 kHz.

**4.** The device according to any one of claims 1 to 3, wherein the impactor (10) has a striking face (11a) for impacting the impact surface (22), an opposite face (11b), opposite to the striking face, and side faces (11c) extending between the striking face (11a) and the opposite face (11b), and wherein said at least one sensor (12) is secured to at least one of the side faces (11c) or to the opposite face.

**5.** The device according to any one of claims 1 to 4, wherein the impactor (10) has a front face (11e) on which a protuberance (9) is formed, the front face (9a) of the protuberance (9) forming the striking face (11a), and wherein the sensor (12) is arranged on a side face of the protuberance (9).

**6.** The device according to any one of claims 1 to 5, wherein the indicator (IN1) corresponds to the average of the deformation, calculated over a time window of programmable duration positioned on the measurement signal.

**7.** The device according to claim 6, wherein the indicator IN1 is calculated as follows:

$$IN1 = \frac{1}{A_1.(t_2 - t_1)} \int_{t_1}^{t_2} A(t).dt$$

where:

A(t) corresponds to the amplitude of the measurement signal at instant t;
$A_1$ corresponds to the maximum amplitude of the measurement signal; and
$t_1$ and $t_2$ correspond respectively to the start and end instants of the time window.

**8.** The device according to any one of claims 1 to 7, wherein the indicator corresponds to the duration of a time window, the start of the time window being defined with respect to an instant corresponding to the first peak of maximum amplitude of the measurement signal and the end of the time window being defined with respect to an instant corresponding to the second peak of maximum amplitude of the measurement signal.

**9.** The device according to any one of claims 1 to 8, further comprising an alert system (33) connected to the processing unit (30) and interacting with the latter so as to emit an alert signal when the indicator converges to a stationary value during successive impacts, or when the indicator exceeds a predetermined threshold value.

**10.** The device according to any one of claims 1 to 9, further comprising an ancillary tool (20) having a rear end forming the impact surface and a front end adapted for cooperating with the implant, the impactor (10) exerting the impact force on the implant via the ancillary tool (20).

11. The device according to any one of claims 1 to 10, wherein the impactor (10) is a hammer, or equivalent, and comprises a gripping shaft (13) topped by a striking head (11), and wherein the sensor is secured to the striking head (11) such that the deformation of the striking head (11) causes the deformation of the sensor (12).

12. The device according to claim 11 , wherein the striking head (11) has a striking face (11a) adapted for impacting the impact surface (22), an opposite face (11b), opposite to the striking face (11a), and side faces (11c) extending between the striking face (11a) and the opposite face (11b), and wherein the sensor (12) is positioned on one of the side faces (11c) extending substantially parallel to the direction of an axis of the gripping shaft (13) and, more specifically, on a front part of the side face (11c), between the striking face (11a) and the axis of the gripping shaft (13).

13. An assembly comprising a device according to any one of claims 1 to 12 and a surgical implant, in particular a femoral stem or an acetabular cup (2).

FIG.1

FIG.2

FIG.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3019031
- FR 3019031 A

- US 2015282856 A1